(19) Europäisches Patentamt / European Patent Office / Office européen des brevets

(11) EP 2 490 654 B1

(12) # EUROPÄISCHE PATENTSCHRIFT

(45) Veröffentlichungstag und Bekanntmachung des Hinweises auf die Patenterteilung:
**28.12.2016 Patentblatt 2016/52**

(21) Anmeldenummer: **10766069.8**

(22) Anmeldetag: **21.10.2010**

(51) Int Cl.:
***A61K 8/73*** (2006.01) ***A61Q 5/06*** (2006.01)

(86) Internationale Anmeldenummer:
**PCT/EP2010/065864**

(87) Internationale Veröffentlichungsnummer:
**WO 2011/048180 (28.04.2011 Gazette 2011/17)**

(54) **ZUSAMMENSETZUNGEN ZUR FORMGEBUNG KERATINISCHER FASERN ENTHALTEND MIT PROPYLENOXID MODIFIZIERTE STÄRKE**

COMPOSITION FOR SHAPING KERATIN FIBRES CONTAINING STARCHES MODIFIED WITH PROPYLENE OXIDE

COMPOSITION POUR LA MISE EN FORME DES FIBRES KÉRATINIQUES, CONTENANT UN AMIDON MODIFIÉ PAR DE L'OXYDE DE PROPYLÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.10.2009 DE 102009045925**

(43) Veröffentlichungstag der Anmeldung:
**29.08.2012 Patentblatt 2012/35**

(73) Patentinhaber: **Henkel AG & Co. KGaA**
**40589 Düsseldorf (DE)**

(72) Erfinder:
- **SCHWEINSBERG, Matthias**
  **22769 Hamburg (DE)**
- **RÖNISCH, Ralf**
  **42349 Wuppertal (DE)**
- **SCHRIEFERS, Mathias**
  **41239 Mönchengladbach (DE)**
- **DOGAN, Carine**
  **F-91270 Vigneux sur Seine (FR)**

(56) Entgegenhaltungen:
**EP-A1- 0 659 394** **EP-A2- 0 948 958**
**EP-A2- 0 948 959** **EP-A2- 0 948 960**
**EP-A2- 1 317 916**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kosmetische Mittel zur temporären Umformung keratinischer Fasern, enthaltend in einem kosmetischen Träger mindestens eine mit Propylenoxid modifizierte Stärke, welche ein mittleres Molekulargewicht (Gewichtsmittel) von 100 bis 1000 kDa besitzt.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Die in Mitteln zur temporären Formgebung üblicherweise eingesetzten synthetischen Polymere werden aus entsprechenden synthetisch zugänglichen Monomeren hergestellt. Besagte Monomere werden aus fossilen Stoffen wie beispielsweise Erdöl durch Umwandlung zu den entsprechenden Polymerbausteinen u.a. unter Aufwand von Energie gewonnen.

Im Rahmen eines nachhaltigeren Umganges mit der Natur als Lebensraum und mit den Resourcen bleibt es wünschenswert, für kosmetische Produkte nur solche kosmetische Rohstoffe zu verwenden, die unter möglichst wenig Einsatz von Energie aus so genannten nachwachsenden Rohstoffen zugänglich sind. Allerdings kann eine Mengenreduktion oder gar ein Austausch besagter synthetischer Polymere nur dann vorgenommen werden, wenn der Ersatz die für den Anwendungszweck gewünschten Eigenschaften aufweist und den keratinhaltigen Fasern einen ausreichenden, stabilen Halt Form gewährleist.

Desweiteren sollen die Ersatzpolymere auf natürlicher Basis die Sprungkraft und Geschmeidigkeit der in der Form fixierten, keratinhaltigen Fasern erhalten. Die Bildung von mit dem bloßen Auge sichtbaren Polymerpartikeln auf den keratinhaltigen Fasern muss vermieden werden. Ferner darf die keratinhaltige Faser nicht stumpf wirken, sondern soll natürlich glänzen.

Aufgabe der vorliegenden Erfindung war es daher, eine formfixierend wirkende, kosmetische Zusammensetzung bereitzustellen, die vorwiegend bis vollständig auf nachwachsenden Rohstoffen basiert, die eine verbesserte Formfixierung bewirkt und die vorgenannten Nachteile nicht aufweist. Dabei soll möglichst ganz auf den Einsatz der auf fossilen Rohstoffen basierenden, synthetischen Polymere verzichtet werden können.

Ein erster Gegenstand der Erfindung ist daher ein kosmetisches Mittel zur temporären Umformung keratinischer Fasern, insbesondere menschlicher Haare, enthaltend in einem kosmetischen Träger mindestens eine mit Propylenoxid modifizierte Stärke, welche ein mittleres Molekulargewicht (Gewichtsmittel) von 100 bis 1000 kDa besitzt. Diese Mittel weisen hervorragende Parameter in der Anwendung am Haar auf. Die besagte Stärke lässt sich nahezu ohne Einsatz von Wärme bei höchstens 30°C durch bloßes Mischen in die erfindungsgemäßen Mittel einarbeiten.

Unter keratinischen Fasern sind dabei erfindungsgemäß Pelze, Wolle, Federn und insbesondere menschliche Haare zu verstehen.

Stärke ist ein Reservekohlenhydrat, das von vielen Pflanzen in Form von üblicherweise 1 bis 200 μm großen Stärkekörnern (Granula) in verschiedenen Pflanzenteilen gespeichert wird, z.B. in Knollen oder Wurzeln, Getreidesamen, Früchten sowie im Mark. Eine erfindungsgemäß verwendbare mit Propylenoxid modifizierte Stärke kann sich aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok ableiten. Besonders ausgeprägte erfindungsgemäße Effekte werden durch mit Propylenoxid modifizierte Tapioka Stärke bzw. mit Propylenoxid modifizierte Kartoffelstärke bzw. durch Gemische beider vorgenannter Stärken, erzielt. Ganz besonders bevorzugt enthält das erfindungsgemäße Mittel mindestens eine mit Propylenoxid modifizierte Kartoffelstärke.

Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten 0 bis 45 Gew.-% Amylose bezogen auf die Trockensubstanz.

Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion.

Es ist erfindungsgemäß bevorzugt, wenn die mit Propylenoxid modifizierte Stärke einen Amylosegehalt von kleiner 25 Gew.-%, insbesondere von kleiner 20 Gew.-%, - jeweils bezogen auf das Gewischt der besagten Stärke - besitzt. Es zeigte sich, dass sich zur Erreichung des erfindungsgemäßen Effektes besonders eine Stärke eignet, die 17

bis 22 Gew.-% Amylose und 78 bis 83 Gew.-% Amylopektin enthält.

Amylose besteht aus überwiegend linear α-1,4-glycosidisch verknüpfter d-Glucose, $M_r$ 50000-150000. Die resultierenden Ketten bilden in der Stärke Doppelhelices.

Amylopektin enthält neben den für Amylose beschriebenen α-1,4-Verknüpfungen auch in einer Menge von 4 bis 6% α-1,6-Bindungen als Verzweigungsstellen. Der durchschnittliche Abstand zwischen den Verzweigungsstellen beträgt etwa 12 bis 17 Glucose-Einheiten. Die Molmasse von $10^7$ bis $7 \cdot 10^8$ entspricht ca. $10^5$ Glucose-Einheiten, womit Amylopektin zu den größten Biopolymeren gehört. Besagte Verzweigungen sind derart über das Molekül verteilt, daß sich eine Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Unter einer mit Propylenoxid modifizierten Stärke wird erfindungsgemäß ein Reaktionsprodukt einer Stärke mit Propylenoxid verstanden. Ein solches Reaktionsprodukt umfasst mindestens eine Struktureinheit der Formel (PS),

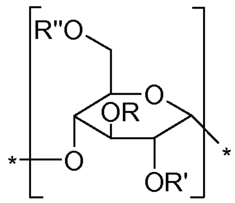

(PS),

worin mindestens ein Rest R, R' oder R" für eine Gruppe der Formel

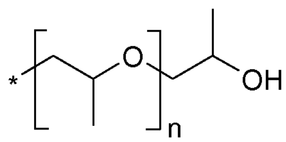

mit n ≥0 steht und maximal 2 der Reste aus R, R', R" für ein Wasserstoffatom steht. Eine mit dem Symbol * gekennzeichnete Bindung in Formeln dieser Anmeldung entspricht einer freien Valenz der entsprechenden Struktureinheit. Die mit Propylenoxid modifizierten Stärken werden beispielsweise durch Umsetzung einer nativen Stärke mit Propylenoxid bereitgestellt. Vor der Modifikation mittels Propylenoxid kann die Stärke diversen physikalischen oder chemischen Prozessen ausgesetzt worden sein, wie z.B. einer Wärmebehandlung, Scherung, einer thermischen, säurehydrolytischen, oxidativen oder enzymatischen Spaltung, etc.

Es ist erfindungsgemäß bevorzugt, wenn die mit Propylenoxid modifizierte Stärke in dem erfindungsgemäßen Mittel nicht in Form einzelner Stärkekörnchen (Granula) vorliegt. Zu diesem Zweck werden die Stärkekörner aufgeschlossen z.B. durch Hitze oder Scherung und die entsprechenden Polysaccharidmoleküle aus dem Verbund freigesetzt. Die freigesetzten Polysaccharidmoleküle werden nach oder vor der Freisetzung mit Propylenoxid modifiziert.

Im Rahmen einer bevorzugten Ausführungsform ist die mit Propylenoxid modifizierte Stärke verkleistert. Wird eine wässrige Suspension von Stärke erhitzt oder komprimiert, so beobachtet man bei einer kritischen Temperatur bzw. Druck eine tangentiale Quellung der Körper mit Verlust der Doppelbrechung, Änderung der Röntgenstruktur und abrupter Steigerung der Viskosität der Lösung. Diese Erscheinung wird Verkleisterung genannt.

Die erfindungsgemäßen mit Propylenoxid modifizierten Stärken liegen in dem erfindungsgemäßen Mittel in einer Molekulargewichtsverteilung vor. Die Molekulargewichtsverteilung wurde experimentell mittels Gelfiltrationschromatographie gegen Dextran bestimmt. Ein wichtiges Merkmal der Erfindung ist das Gewichtsmittel des mittleren Molekulargewichts der in dem erfindungsgemäßen Mittel enthaltenen Propylenoxid modifizierten Stärken. Das besagte Gewichtsmittel ist ein mittleres Molekulargewicht, welches das Totalgewicht der Moleküle verschiedenen Molekulargewichts berücksichtigt und nicht lediglich nur die Anzahl der Moleküle. Zur statistischen Berechnung des Gewichtsmittels wird zunächst der "Gewichtsbruch"

$$w_i = (N_i M_i) / [\Sigma(N_i M_i)]$$

definiert. Dieser gibt den Gewichtsanteil an Makromolekülen in der Probe an, die aus *i* Segmenten (z. B. Monomerbausteinen) der Masse $M_i$ bestehen und in der Probe $N_i$-mal vorkommen. Für das Gewichtsmittel des Molekulargewichts $M_w = \Sigma w_i M_i$ gilt damit

$$M_w = [\Sigma(N_i M^2_i)] / [\Sigma(N_i M_i)].$$

Bevorzugte erfindungsgemäße Mittel enthalten solche mit Propylenoxid modifizierten Stärken, die ein mittleres Molekulargewicht (Gewichtsmittel) von 100 bis 400 kDa, bevorzugt von 200 bis 300 kDa, aufweisen. Die erfindungsgemäßen Mittel dieser Ausführungsform sind bevorzugt in Schäume oder Gele als kosmetischer Träger eingearbeitet und liegen daher als Schaum oder Gel vor. Trotz des für festigende Polymere geringen Molekulargewichts lässt sich mit diesen besagten Stärken eine hervorragende Festigung erzielen. Ferner erreichen die mit besagten Stärken hergestellten Gele zusätzlich eine superbe Transparenz.

Ferner eignen sich mit Propylenoxid modifizierte Stärken (insbesondere mit Propylenoxid modifizierte Kartoffelstärken) besonders bevorzugt für den Einsatz in den erfindungsgemäßen Mitteln, wenn sie ein Molekulargewicht (Gewichtmittel) von 100 bis 1000 kDa, insbesondere von 700 bis 900 kDa, aufweisen. Die erfindungsgemäßen Mittel dieser Ausführungsform sind bevorzugt in Schäume oder Gele als kosmetischer Träger eingearbeitet und liegen daher als Schaum oder Gel vor. Trotz des für festigende Polymere geringen Molekulargewichts lässt sich mit diesen besagten Stärken eine hervorragende Festigung erzielen. Ferner erreichen die mit besagten Stärken hergestellten Gele zusätzlich eine superbe Transparenz.

Zur Einstellung des Molekulargewichts wird die Stärke vor oder nach der Modifizierung mit Propylenoxid einer mechanischen und/oder einer chemischen Behandlung unterzogen. Zur Molekulargewichtserhöhung kann die besagte Stärke vernetzt werden. Eine Vernetzung der mit Propylenoxid modifizierten Stärke liegt vor, wenn die linearen bzw. verzweigten Polysaccharid-Makromoleküle der Stärke kovalent durch ein Vernetzungsmittel unter Bildung eines dreidimensionalen, unlöslichen und nur noch quellbaren polymeren Netzwerkes verknüpft werden. Native Stärke gilt im Allgemeinen als unvernetzt und bedarf-falls eine Vernetzung gewünscht wäre - einer künstlichen Vernetzung mittels Synthesechemie. Eine solche künstliche Vernetzung lässt sich mit Vernetzungsmitteln durchführen. (Mit Propylenoxid modifizierte) Stärken, die eine solche Vernetzung nicht aufweisen, sind unvernetzt.

Eine Vernetzung erfolgt beispielsweise durch das Vernetzungmittel Epichlorhydrin. Hierzu wird eine 42 Gew.-%-ige Mischung von mit Propylenoxid modifizierter Stärke in Wasser hergestellt, in diese die gewünschte Menge Epichlorhydrin bei Raumtemperatur eingerührt und nach einer Rührzeit von 1 bis 5 Stunden unter Kontrolle der Viskosität nach erreichen der Zielviskosität die vernetzte Stärke nach gängigen Verfahren isoliert.

Es ist jedoch erfindungsgemäß bevorzugt, wenn die in den erfindungsgemäßen Mitteln enthaltene(n) mit Propylenoxid modifizierte(n) Stärke(n) unvernetzt sind.

Zur Erzielung eines niedrigeren Molekulargewichts von 100 bis 400 kDa, bzw. 200 bis 300 kDa, werden besagte Stärken bevorzugt einer mechanischen Spaltung, einer enzymatischen Spaltung (insbesondere mit alpha-Amylase, beta-Amylase, Glucoamylase oder entzweigende Enzyme), einer säurehydrolytischen Spaltung (insbesondere mit Salzsäure, Schwefelsäure oder Phosphorsäure), einer thermischen Spaltung oder einer Umsetzung mit Oxidationsmitteln (wie Periodat, Hypochlorit, Chromsäure, Permanganat, Stickstoffdioxid, Wasserstoffperoxid oder organischer Percarbonsäure, bevorzugt mit Wasserstoffperoxid), ausgesetzt. Zur mechanischen Spaltung der Stärke eignen sich Kneter, extruder, Stator/Rotor-Maschinen und/oder Rührwerke.

Die oxidative Spaltung mittels Wasserstoffperoxid ist erfindungsgemäß bevorzugt. Zu diesem Zweck wird beispielsweise die mit Propylenoxid modifizierte Stärke in Wasser gegeben, auf 50 bis 70 °C erhitzt, Wasserstoffperoxid zugefügt und bei 70 bis 85°C für 2 bis 5 Stunden gerührt.

Der Gehalt an Propylenoxid der Stärke wirkt sich auf die Feinabstimmung des Frisurenhalts mit der Frisurenflexibilität und der Stabilität der kosmetischen Mittel aus. Es zeigte sich, dass die Parameter weiter optimiert werden, wenn die mit Propylenoxid modifizierte Stärke - bezogen auf das Gewicht der besagten Stärke - einen Propylenoxidgehalt von 1 bis 20 Gew.-%, besonders bevorzugt einen Propylenoxidgehalt von 8 bis 12 Gew.-%, ganz besonders bevorzugt einen Propylenoxidgehalt von 9,5 bis 10,5 Gew.-%, aufweist. Im Rahmen einer weiteren Ausführungsform kann es bevorzugt sein, wenn die mit Propylenoxid modifizierte Stärke - bezogen auf das Gewicht der besagten Stärke - einen Propylenoxidgehalt von 1 bis 20 Gew.-%, besonders bevorzugt einen Propylenoxidgehalt von 3 bis 10 Gew.-%, ganz besonders bevorzugt einen Propylenoxidgehalt von 4,0 bis 6,0 Gew.-%, aufweist. Der Propylenoxidgehalt lässt sich beispielsweise nach Durchführung einer Hodges-Spaltung mit der Methode nach DIN EN 13268 bestimmen.

Ferner hat es sich erwiesen, dass solche kosmetischen Mittel sich im Sinne der Erfindung hervorragend eignen, in denen die mit Propylenoxid modifizierte Stärke in einer 43 Gew.-%-igen wässrigen Lösung eine bevorzugte Viskosität im Bereich von 150 bis 1500000 mPa·s (Brookfield Viskosimeter, Spindel #7 bei 20°C und 20 U/min) aufweist. Hervorragend geeignete Propylenoxid modifizierte Polysaccharide weisen Viskositäten von 3000 bis 80000 mPa·s, insbesondere von 10000 bis 100000 mPa·s, besonders bevorzugt von 40000 bis 70000 mPa·s, im Rahmen einer speziellen Ausführungsform besonders bevorzugt von 5000 bis 8000 mPa·s, (jeweils gemessen unter den zuvor genannten Bedingungen) auf.

Es ist erfindungsgemäß bevorzugt, wenn das kosmetisches Mittel das mit Propylenoxid modifizierte Polysaccharid in einer Menge von 1,0 Gew.-% bis 30 Gew.-%, insbesondere von 2,5 Gew.-% bis 20 Gew.-%, jeweils bezogen auf das Gewicht des Mittels, enthält.

Das erfindungsgemäße kosmetische Mittel ist bevorzugt frei von synthetischen, filmbildenden Polymeren und synthetischen, festigenden Polymeren, die jeweils auf Basis von fossilen Rohstoffen hergestellt wurden. Unter fossilen Rohstoffen werden erfindungsgemäß solche Stoffe verstanden, deren Ursprung sich aus fossilen Brennstoffen herleitet.

Unter Polymeren werden erfindungsgemäß Verbindungen verstanden, die aus einer Vielzahl von Molekülen aufgebaut sind, in denen eine Art oder mehrere Arten von Atomen oder Atomgruppierungen (sogenannte konstitutive Einheiten, Grundbausteine oder Wiederholungseinheiten) wiederholt aneinander gereiht sind und die ein Molekulargewicht von wenigstens 10000 g/mol besitzen. Die Polymere werden durch Polyreaktion erhalten, wobei letztere künstlich (d.h. synthetisch) oder natürlich erfolgen kann.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser, Alkohol oder Wasser/Alkohol-Gemischen besitzen. So lassen sich entsprechende Lösungen herstellen, die sich auf einfache Art und Weise anwenden bzw. weiterverarbeiten lassen.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nichtionisch, permanent kationisch oder temporär kationisch geladen sein.

Es sind somit erfindungsgemäß solche kosmetische Mittel bevorzugt, die weiterhin mindestens ein zusätzliches Polymer enthalten, mit der Maßgabe, dass alle zusätzlichen Polymere Polymere auf Polysacharidbasis sind. Diese zusätzlichen Polymere sind selbstredend von den Propylenoxid modifizierten Polysacchariden verschieden. Wiederum bevorzugt werden alle weiteren Polymere des erfindungsgemäßen kosmetischen Mittels ausgewählt aus Xanthan, Dehydroxanthan, Alginat, Guar Gum, Gummi Arabicum, Locust Bean Gum, Stärke, Chitosan oder Gemischen.

Die zusätzlichen Polymere sind bevorzugt in einer Menge von 0,5 Gew.-% bis 30 Gew.-%, insbesondere von 2,5 Gew.-% bis 20 Gew.-%, jeweils bezogen auf das Gewicht des Mittels, enthalten.

Erfindungsgemäß ganz besonders bevorzugte kosmetische Mittel gehorchen mindestens einer der folgenden Ausführungsformen A) bis W):

A):

Kosmetisches Mittel zur temporären Umformung keratinischer Fasern, insbesondere menschlicher Haare, enthaltend in einem kosmetischen Träger mindestens eine mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa,
bevorzugt von 100 bis 400 kDa, ganz besonders bevorzugt von 200 bis 300 kDa, welche - bezogen auf das Gewicht besagter Stärke - einen Propylenoxidgehalt von 5 bis 15 Gew.-% aufweist.

B):

Kosmetisches Mittel zur temporären Umformung keratinischer Fasern, insbesondere menschlicher Haare, enthaltend in einem kosmetischen Träger mindestens eine mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa,
bevorzugt von 100 bis 400 kDa, ganz besonders bevorzugt von 200 bis 300 kDa, welche - bezogen auf das Gewicht besagter Stärke - einen Propylenoxidgehalt von 8,0 bis 12,0 Gew.-% aufweist.

C):

Kosmetisches Mittel zur temporären Umformung keratinischer Fasern, insbesondere menschlicher Haare, enthaltend in einem kosmetischen Träger mindestens eine mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa,
bevorzugt von 100 bis 400 kDa, ganz besonders bevorzugt von 200 bis 300 kDa, welche - bezogen auf das Gewicht besagter Stärke - einen Propylenoxidgehalt von 9,5 bis 10,5 Gew.-% aufweist.

D):

Kosmetisches Mittel zur temporären Umformung keratinischer Fasern, insbesondere menschlicher Haare, enthaltend in einem kosmetischen Träger
(a) mindestens eine mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 400 kDa, ganz besonders bevorzugt von 200 bis 300 kDa, welche - bezogen auf das Gewicht besagter Stärke - einen Propylenoxidgehalt von 5 bis 15 Gew.-% aufweist und
(b) mindestens ein zusätzliches Polymer, mit der Maßgabe, dass alle zusätzlichen Polymere Polymere auf Polysacharidbasis sind.

E):

Kosmetisches Mittel zur temporären Umformung keratinischer Fasern, insbesondere menschlicher Haare, enthaltend in einem kosmetischen Träger

(a) mindestens eine mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 400 kDa, ganz besonders bevorzugt von 200 bis 300 kDa, welche - bezogen auf das Gewicht besagter Stärke - einen Propylenoxidgehalt von 8 bis 12 Gew.-% aufweist und
(b) mindestens ein zusätzliches Polymer, mit der Maßgabe, dass alle zusätzlichen Polymere Polymere auf Polysacharidbasis sind.

F):

Kosmetisches Mittel zur temporären Umformung keratinischer Fasern, insbesondere menschlicher Haare, enthaltend in einem kosmetischen Träger

(a) mindestens eine mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 400 kDa, ganz besonders bevorzugt von 200 bis 300 kDa, welche - bezogen auf das Gewicht besagter Stärke - einen Propylenoxidgehalt von 9,5 bis 10,5 Gew.-% aufweist und
(b) mindestens ein zusätzliches Polymer, mit der Maßgabe, dass alle zusätzlichen Polymere Polymere auf Polysacharidbasis sind.

G):

Kosmetisches Mittel zur temporären Umformung keratinischer Fasern, insbesondere menschlicher Haare, enthaltend in einem kosmetischen Träger mindestens eine mit Propylenoxid modifizierte Stärke, in einer Menge von 2,5 Gew.-% bis 20 Gew.-%, bezogen auf das Gewicht des Mittels welche mit Wasserstoffperoxid degradiert wurde und ein mittleres Molekulargewicht (Gewichtsmittel) von 100 bis 400 kDa, ganz besonders bevorzugt von 200 bis 300 kDa sowie - bezogen auf das Gewicht besagter Stärke - einen Propylenoxidgehalt von 5 bis 15 Gew.-% aufweist.

H):

Kosmetisches Mittel zur temporären Umformung keratinischer Fasern, insbesondere menschlicher Haare, enthaltend in einem kosmetischen Träger mindestens eine mit Propylenoxid modifizierte Stärke in einer Menge von 2,5 Gew.-% bis 20 Gew.-%, bezogen auf das Gewicht des Mittels welche mit Wasserstoffperoxid degradiert wurde und ein mittleres Molekulargewicht (Gewichtsmittel) von 100 bis 400 kDa, ganz besonders bevorzugt von 200 bis 300 kDa sowie - bezogen auf das Gewicht besagter Stärke - einen Propylenoxidgehalt von 8 bis 12 Gew.-% aufweist.

I):

Kosmetisches Mittel zur temporären Umformung keratinischer Fasern, insbesondere menschlicher Haare, enthaltend in einem kosmetischen Träger mindestens eine mit Propylenoxid modifizierte Stärke in einer Menge von 2,5 Gew.-% bis 20 Gew.-%, bezogen auf das Gewicht des Mittels welche mit Wasserstoffperoxid degradiert wurde und ein mittleres Molekulargewicht (Gewichtsmittel) von 100 bis 400 kDa, ganz besonders bevorzugt von 200 bis 300 kDa sowie - bezogen auf das Gewicht besagter Stärke - einen Propylenoxidgehalt von 9,5 bis

10,5 Gew.-% aufweist.

J):

Kosmetisches Mittel zur temporären Umformung keratinischer Fasern, insbesondere menschlicher Haare, enthaltend in einem kosmetischen Träger mindestens eine unvernetzte, mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa, bevorzugt von 700 bis 900 kDa, welche - bezogen auf das Gewicht besagter Stärke - einen Propylenoxidgehalt von 5 bis 15 Gew.-% aufweist.

K):

Kosmetisches Mittel zur temporären Umformung keratinischer Fasern, insbesondere menschlicher Haare, enthaltend in einem kosmetischen Träger mindestens eine unvernetzte, mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa, bevorzugt von 700 bis 900 kDa, welche - bezogen auf das Gewicht besagter Stärke - einen Propylenoxidgehalt von 8,0 bis 12,0 Gew.-% aufweist.

L):

Kosmetisches Mittel zur temporären Umformung keratinischer Fasern, insbesondere menschlicher Haare, enthaltend in einem kosmetischen Träger mindestens eine unvernetzte, mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa, bevorzugt von 700 bis 900 kDa, welche - bezogen auf das Gewicht besagter Stärke - einen Propylenoxidgehalt von 9,5 bis 10,5 Gew.-% aufweist.

M):

Kosmetisches Mittel zur temporären Umformung keratinischer Fasern, insbesondere menschlicher Haare, enthaltend in einem kosmetischen Träger mindestens eine mit unvernetzte,
mit Propylenoxid modifizierte Stärke, in einer Menge von 2,5 Gew.-% bis 20 Gew.-%, bezogen auf das Gewicht des Mittels welche ein mittleres Molekulargewicht (Gewichtsmittel) von 100 bis 1000 kDa, ganz besonders bevorzugt von 700 bis 900 kDa, eine Viskosität von 10000 bis 100000 m·Pas, bevorzugt von 40000 bis 70000 m·Pas, sowie - bezogen auf das Gewicht besagter Stärke - einen Propylenoxidgehalt von 5 bis 15 Gew.-% aufweist.

N):

Kosmetisches Mittel zur temporären Umformung keratinischer Fasern, insbesondere menschlicher Haare, enthaltend in einem kosmetischen Träger mindestens eine mit unvernetzte,
mit Propylenoxid modifizierte Stärke, in einer Menge von 2,5 Gew.-% bis 20 Gew.-%, bezogen auf das Gewicht des Mittels welche ein mittleres Molekulargewicht (Gewichtsmittel) von 100 bis 1000 kDa, ganz besonders bevorzugt von 700 bis 900 kDa sowie - bezogen auf das Gewicht besagter Stärke - einen Propylenoxidgehalt von 8 bis 12 Gew.-% aufweist.

O):

Kosmetisches Mittel zur temporären Umformung keratinischer Fasern, insbesondere menschlicher Haare, enthaltend in einem kosmetischen Träger mindestens eine mit unvernetzte,
mit Propylenoxid modifizierte Stärke, in einer Menge von 2,5 Gew.-% bis 20 Gew.-%, bezogen auf das Gewicht des Mittels welche ein mittleres Molekulargewicht (Gewichtsmittel) von 100 bis 1000 kDa, ganz besonders bevorzugt von 700 bis 900 kDa sowie - bezogen auf das Gewicht besagter Stärke - einen Propylenoxidgehalt von 9,5 bis 10,5 Gew.-% aufweist.

P):

Kosmetisches Mittel zur temporären Umformung keratinischer Fasern, insbesondere menschlicher Haare, enthaltend in einem kosmetischen Träger

(a) mindestens eine unvernetzte, mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa, ganz besonders bevorzugt von 700 bis 900 kDa, welche - bezogen auf das Gewicht besagter Stärke - einen Propylenoxidgehalt von 5 bis 15 Gew.-% aufweist und

(b) mindestens ein zusätzliches Polymer, mit der Maßgabe, dass alle zusätzlichen Polymere Polymere auf Polysacharidbasis sind.

Q):

Kosmetisches Mittel zur temporären Umformung keratinischer Fasern, insbesondere menschlicher Haare, enthaltend in einem kosmetischen Träger

(a) mindestens eine unvernetzte, mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa, ganz besonders bevorzugt von 700 bis 900 kDa, welche - bezogen auf das Gewicht besagter Stärke - einen Propylenoxidgehalt von 8 bis 12 Gew.-% aufweist und
(b) mindestens ein zusätzliches Polymer, mit der Maßgabe, dass alle zusätzlichen Polymere Polymere auf Polysacharidbasis sind.

R):

Kosmetisches Mittel zur temporären Umformung keratinischer Fasern, insbesondere menschlicher Haare, enthaltend in einem kosmetischen Träger

(a) mindestens eine unvernetzte, mit Propylenoxid modifizierte Stärke in einer Menge von 2,5 Gew.-% bis 20 Gew.-%, bezogen auf das Gewicht des Mittels mit einem mittleren Molekulargewicht (Gewichtsmittel) von 100 bis 1000 kDa, ganz besonders bevorzugt von 700 bis 900 kDa, welche - bezogen auf das Gewicht besagter Stärke - einen Propylenoxidgehalt von 9,5 bis 10,5 Gew.-% aufweist und
(b) mindestens ein zusätzliches Polymer, mit der Maßgabe, dass alle zusätzlichen Polymere Polymere auf Polysacharidbasis sind.

S):

Kosmetisches Mittel zur temporären Umformung keratinischer Fasern, insbesondere menschlicher Haare, enthaltend in einem kosmetischen Träger mindestens eine mit unvernetzte, mit Propylenoxid modifizierte Kartoffelstärke, in einer Menge von 2,5 Gew.-% bis 20 Gew.-%, bezogen auf das Gewicht des Mittels welche ein mittleres Molekulargewicht (Gewichtsmittel) von 100 bis 1000 kDa, ganz besonders bevorzugt von 700 bis 900 kDa sowie - bezogen auf das Gewicht besagter Stärke - einen Propylenoxidgehalt von 5 bis 15 Gew.-% aufweist.

T):

Kosmetisches Mittel zur temporären Umformung keratinischer Fasern, insbesondere menschlicher Haare, enthaltend in einem kosmetischen Träger mindestens eine mit unvernetzte, mit Propylenoxid modifizierte Kartoffelstärke, in einer Menge von 2,5 Gew.-% bis 20 Gew.-%, bezogen auf das Gewicht des Mittels welche ein mittleres Molekulargewicht (Gewichtsmittel) von 100 bis 1000 kDa, ganz besonders bevorzugt von 700 bis 900 kDa sowie - bezogen auf das Gewicht besagter Stärke - einen Propylenoxidgehalt von 8 bis 12 Gew.-% aufweist.

U):

Kosmetisches Mittel zur temporären Umformung keratinischer Fasern, insbesondere menschlicher Haare, enthaltend in einem kosmetischen Träger mindestens eine mit unvernetzte, mit Propylenoxid modifizierte Kartoffelstärke, in einer Menge von 2,5 Gew.-% bis 20 Gew.-%, bezogen auf das Gewicht des Mittels welche ein mittleres Molekulargewicht (Gewichtsmittel) von 100 bis 1000 kDa, ganz besonders bevorzugt von 700 bis 900 kDa, eine Viskosität von 10000 bis 100000 m·Pas, bevorzugt von 40000 bis 70000 m·Pas, sowie - bezogen auf das Gewicht besagter Stärke - einen Propylenoxidgehalt von 5 bis 15 Gew.-% aufweist.

V):

Kosmetisches Mittel zur temporären Umformung keratinischer Fasern, insbesondere menschlicher Haare, enthaltend in einem kosmetischen Träger mindestens eine mit unvernetzte, mit Propylenoxid modifizierte Kartoffelstärke, in einer Menge von 2,5 Gew.-% bis 20 Gew.-%, bezogen auf das Gewicht des Mittels welche ein mittleres Molekulargewicht (Gewichtsmittel) von 100 bis 1000 kDa, ganz besonders bevorzugt von 700 bis 900 kDa, eine Viskosität von 10000 bis 100000 m·Pas, bevorzugt von 40000 bis 70000 m·Pas, sowie - bezogen auf das Gewicht besagter Stärke - einen Propylenoxidgehalt von 8 bis 12 Gew.-% aufweist.

W):

Kosmetisches Mittel zur temporären Umformung keratinischer Fasern, insbesondere menschlicher Haare, enthaltend in einem kosmetischen Träger mindestens eine mit unvernetzte, mit Propylenoxid modifizierte Kartoffelstärke, in einer Menge von 2,5 Gew.-% bis 20 Gew.-%, bezogen auf das Gewicht des Mittels welche ein mittleres Molekulargewicht (Gewichtsmittel) von 100 bis 1000 kDa, ganz besonders bevorzugt von 700 bis 900 kDa, eine Viskosität von 10000 bis 100000 m·Pas, bevorzugt von 40000 bis 70000 m·Pas, sowie - bezogen auf das Gewicht besagter Stärke - einen Propylenoxidgehalt von 9,5 bis 10,5 Gew.-% aufweist.

Die Viskositäten der Ausführungsformen A bis W - wenn aufgeführt - werden gemäß der zuvor genannten Messbedingungen definiert.

Im Rahmen der zuvor genannten Ausführungsformen gelten selbstverständlich *mutatis mutandis* die jeweiligen als bevorzugt gekennzeichneten Merkmale des erfindungsgemäßen Mittels, wie insbesondere die Einsatzmengen.

Zur Verbesserung der erfindungsgemäßen Effekte eignet sich bevorzugt der weitere Zusatz mindestens einer Verbindung der Formel (I),

$$HO\text{-}CH_2\text{-}(CHOH)_n\text{-}CH_2\text{-}OH \qquad (I)$$

worin n eine ganze Zahl von 1 bis 4 bedeutet.

Besonders effektvolle zeigen sich die erfindungsgemäßen Mittel, wenn sie als Verbindungen der Formel (I) Glycerin und/oder Sorbitol enthalten.

Ferner erwies sich eine Einsatzmenge der Verbindungen der Formel (I) im Bereich von 0,2 bis 10 Gew.-%, insbesondere von 0,5 bis 7 Gew.-% als vorteilhaft.

Es hat sich als bevorzugt herausgestellt, zusätzlich mindestens ein nichtionisches Tensid einzusetzen. Diese Tenside können erfindungsgemäß bereits emulgierende Wirkung haben.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 100 Mol Ethylenoxid und/oder 1 bis 5 Mol Propylenoxid an lineare oder verzweigte Fettalkohole mit 8 bis 30 Kohlenstoff-Atomen, an Fettsäuren mit 8 bis 30 Kohlenstoff-Atomen und an Alkylphenole mit 8 bis 15 C-Atomen in der Alkylgruppe,
- Anlagerungsprodukte von 2 bis 20 Einheiten Glyzerin an lineare oder verzweigte Fettalkohole mit 8 bis 30 Kohlenstoff-Atomen in der Alkylgruppe, an lineare oder verzweigte Fettsäuren mit 8 bis 30 Kohlenstoff-Atomen in der Alkylgruppe, wie beispielsweise die unter den Verkaufsbezeichnungen Dermofeel® G 10 LW (Straetmans Chemische Produkte) erhältlichen Typen,
- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 Kohlenstoff -Atomen, an Fettsäuren mit 8 bis 30 C-Atomen und an Alkylphenole mit 8 bis 15 Kohlenstoff -Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO\text{-}(OCH_2CHR^2)_wOR3 \qquad (E4\text{-}I)$$

in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,

- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$$R^4O\text{-}[G]_p \qquad (E4\text{-}II)$$

in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkylolioglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest $R^4$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkyloligoglucoside auf Basis technischer $C_{9/11}$-Oxoalkohole (DP = 1 bis 3). Der Alkyl- bzw. Alkenylrest $R^{15}$ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

Darunter sind wiederum solche nichtionischen Tenside besonders bevorzugt zum Einsatz in dem erfindungsgemäßen Mittel geeignet, die ausgewählt werden aus

- Anlagerungsprodukten von 2 bis 20 Einheiten Glyzerin an lineare oder verzweigte Fettalkohole mit 8 bis 30 Kohlenstoff-Atomen in der Alkylgruppe,
- Anlagerungsprodukten von 2 bis 20 Einheiten Glyzerin an lineare oder verzweigte Fettsäuren mit 8 bis 30 Kohlenstoff-Atomen in der Alkylgruppe,
- Anlagerungsprodukte von 5 bis 60 Mol Ethylenoxid an Rizinusöl und gehärtetes Rizinusöl,
- Zuckertensiden vom Typ der Alkyl- und Alkenyloligoglykoside gemäß obiger Formel (E4-II),
- Mischungen aus den vorgenannten Tensiden.

Die nichtionischen Tenside sind bevorzugt in einer Menge von 0,005 Gew.-% bis 10 Gew.-%, insbesondere von 0,01 bis 2 Gew.-%, jeweils bezogen auf das Gewicht des erfindungsgemäßen Mittels, in dem erfindungsgemäßen Mittel enthalten.

Ferner enthalten die erfindungsgemäßen Mittel bevorzugt zusätzlich mindestens ein Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Geeignete Pflanzenextrakte werden durch Extraktion mit organischen Lösemitteln (wie beispielsweise Ethanol, Isopropanol, Diethylether, Benzin, Benzol, Chloroform) oder durch Wasserdampfdestillation gewonnen. Erfindungsgemäß sind vor allem die Extrakte aus Bambus, Leinsamen, Seerose, grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich,

Eibisch, Meristem, Ginseng und Ingwerwurzel bevorzugt.

Der zusätzliche Pflanzenextrakt ist bevorzugt in dem erfindungsgemäßen Mittel in einer Menge von 0,05 Gew.-% bis 1,0 Gew.-%, insbesondere von 0,1 Gew.-% bis 0,5 Gew.-%, jeweils bezogen auf das Gewicht des kosmetischen Mittels, enthalten.

Es ist erfindungsgemäß bevorzugt - insbesondere wenn das erfindungsgemäße Mittel als Creme formuliert ist - dass das erfindungsgemäße kosmetische Mittel zusätzlich mindestens eine Ölphase enthält.

Unter einer Ölphase versteht sich erfindungsgemäß eine bei 20°C flüssige Phase, die sich bei 20°C zu weniger als 1 g in 100 g Wasser löst.

Die Ölphase besitzt bevorzugt eine Viskosität bis zu 1000 mPas, (Brookfield, RVDV II+, 20°C, 20 Umdrehungen pro Minute, Spindel Nr. 1).

In einer bevorzugten Ausführungsform wird das Öl der Ölphase ausgewählt aus mindestens einem Öl der Gruppe, die gebildet wird aus

- pflanzliche Öle,
- tierische Öle,
- Esterölen,
- flüssige Fettsäuren und/oder deren Mono,- Di- und Trifettsäureester von gesättigten und/oder ungesättigten linearen und/oder verzweigten $C_6$- bis $C_{22}$-Fettsäuren mit Glycerin.

Bevorzugte pflanzliche Öle werden ausgewählt aus mindestens einem Vertreter der Gruppe, die gebildet wird aus Amaranthöl, Sonnenblumenöl, Olivenöl, Sojaöl, Rapsöl, Ricinusöl, Sesamöl, Mandelöl, Jojobaöl, Orangenöl, Aprikosenkernöl, Macadamianussöl, Weizenkeimöl, Pfirsichkernöl und die flüssigen Anteile des Kokosöls.

Bevorzugte Esteröle werden ausgewählt aus Estern von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).

Bevorzugt als Öl in der Ölphase verwendbare Mono,- Di- und Trifettsäureester von gesättigten und/oder ungesättigten linearen und/oder verzweigten Fettsäuren mit Glycerin sind die insbesondere Triglyceridester aus Caprinsäure und Caprylsäure (INCI-Bezeichnung: Caprylic/Capric Triglyceride) beispielsweise erhältlich als Handelsprodukt der Firma Cognis unter der Bezeichnung Myritol® 312.

Die zusätzliche Ölphase ist bevorzugt in dem erfindungsgemäßen Mittel in einer Menge von 0,05 Gew.-% bis 25 Gew.-%, insbesondere von 0,1 Gew.-% bis 20 Gew.-%, jeweils bezogen auf das Gewicht des kosmetischen Mittels, enthalten.

Weiterhin eignen sich erfindungsgemäße Mittel, die zusätzlich mindestens einen Fettstoff enthalten.

Unter einem Fettstoff werden erfindungsgemäß solche Verbindungen verstanden, die bei 20°C zu weniger als 1 g in 100 g Wasser löslich sind.

Bevorzugt wird der Fettstoff ausgewählt aus mindestens einem Fettstoff der Gruppe, die gebildet wird aus Candelillawachs, Sheabutter, Carnaubawachs, Bienenwachs, Kokosfett, $C_{12}$ bis $C_{20}$-Fettsäuren (insbesondere Palmitinsäure, Stearinsäure)

Der zusätzliche Fettstoff ist bevorzugt in dem erfindungsgemäßen Mittel in einer Menge von 0,05 Gew.-% bis 35 Gew.-%, insbesondere von 1 Gew.-% bis 20 Gew.-%, jeweils bezogen auf das Gewicht des kosmetischen Mittels,

enthalten.

Das erfindungsgemäße kosmetische Mittel kann - bevorzugt unter Einhaltung der Maxime, nur solche Rohstoffe einzusetzen, die nicht ihren Ursprung in fossilen Brennstoffen haben - weiterhin zusätzliche Hilfs- und Zusatzstoffe enthalten.

Als geeignete Hilfs- und Zusatzstoffe sind insbesondere Pflegestoffe zu nennen.

Die erfindungsgemäßen Mittel enthalten ihre Wirkstoffe in einem kosmetischen Träger, bevorzugt in einem wasserhaltigen kosmetischen Träger, alkoholischen kosmetischen Träger oder einem wässrig-alkoholischen kosmetischen Träger. Zum Zwecke der temporären Haarumformung sind solche Träger beispielsweise Lotionen, Wasser-in-Öl-Emulsionen, Öl-in-Wasser-Emulsionen, Cremes, Gele, Schäume, Pomaden, Wachse oder andere Zubereitungen, die für die Anwendung auf dem Haar geeignet sind.

Unter wässrig-alkoholischen Trägern sind im Sinne der vorliegenden Erfindung wässrige Zusammensetzungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol, 1,2-Propylenglykol oder 1,3-Propylenglykol enthalten. Bevorzugt sind dabei alle wasserlöslichen organischen Lösemittel.

Als Pflegestoff kann ein kationisches Tensid eingesetzt werden. Bevorzugt sind dabei kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Da sich der Zusatz oberflächenaktiver Substanzen jedoch negativ auf die hydrophoben Eigenschaften des hydrophobierten Siliciumdioxids und damit auf die Stabilität des erfindungsgemäßen kosmetischen Mittels auswirken kann, ist die Menge an pflegendem Tensid sorgfältig auf die Gesamtzusammensetzung abzustimmen. Vorzugsweise wird auf den Zusatz tensidischer Bestandteile verzichtet.

Als Pflegestoff eignen sich ebenfalls pflegende Polymere.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein mittleres Molekulargewicht (Gewichtsmittel) von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17$^{th}$ Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat

Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Bevorzugt sind die kationischen Proteinhydrolysate und -derivate auf pflanzlicher Basis.

Weitere erfindungsgemäß einsetzbare pflegende Polymere sind amphotere Polymere.

Als Pflegestoff kann weiterhin mindestens ein Vitamin, ein Provitamin, eine Vitaminvorstufe und/oder eines derer Derivate eingesetzt werden.

Dabei sind erfindungsgemäß solche Vitamine, Pro-Vitamine und Vitaminvorstufen bevorzugt, die üblicherweise den Gruppen A, B, C, E, F und H zugeordnet werden. Besonders bevorzugt sind Vitamine, die zur B-Gruppe oder zu dem Vitamin B-Komplex gehören, ganz besonders bevorzugt Vitamin $B_5$ (Pantothensäure, Panthenol und Pantolacton).

Als Pflegestoff eignen sich weiterhin eine Reihe von Carbonsäuren.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Weitere geeignete Pflegestoffe sind Proteinhydrolysate und/oder deren Derivate, wobei die Verwendung von Proteinhydrolysaten pflanzlichen Ursprungs, z. B. Soja-, Mandel-, Erbsen-, Kartoffel- und Weizenproteinhydrolysaten, bevorzugt ist. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Lexein® (Inolex), Crolastin® (Croda), Crosilk® (Croda) oder Crotein® (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Weiterhin sind als Pflegestoff Lipide und Ölkörper, beispielsweise pflanzliche Öle, flüssige Paraffinöle, Isoparaffinöle, synthetische Kohlenwasserstoffe und Esteröle, Enzyme und Perlenextrakte geeignet.

Neben den Pflegestoffen können auch weitere Hilfs- und Zusatzstoffe zugegeben werden.

Durch Zugabe eines UV-Filters können sowohl die Zubereitungen selbst, als auch die behandelten Fasern vor schädlichen Einflüssen von UV-Strahlung geschützt werden. Es kann daher vorteilhaft sein, den erfindungsgemäßen kosmetischen Mitteln zusätzlich mindestens einen UV-Filter zuzugeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Beispielhaft sei hier 2-Hydroxy-4-methoxybenzophenon-5-sulfonsäure und deren Natriumsalz (Benzophenone-4; Uvinul®MS 40; Uvasorb®S 5) genannt.

In einer besonderen Ausführungsform enthält das erfindungsgemäße kosmetische Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Weiterhin können die erfindungsgemäßen kosmetischen Mittel Alkalisierungsmittel, üblicherweise Alkali- oder Erdalkalihydroxide, Ammoniak oder organische Amine, enthalten. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Ein zweiter Gegenstand der Erfindung ist die Verwendung eines kosmetischen Mittels des ersten Erfindungsgegenstandes zur temporären Umformung und/oder Formfixierung keratinischer Fasern, insbesondere menschlicher Haare.

Ein dritter Gegenstand der Erfindung ist ein Verfahren zur temporären Umformung keratinischer Fasern, insbesondere menschlicher Haare, dadurch gekennzeichnet, dass ein kosmetisches Mittel des ersten Erfindungsgegenstandes auf die keratinischen Fasern aufgebracht wird.

Es hat sich als bevorzugt herausgestellt, wenn die keratinischen Fasern nach der Einwirkung der kosmetischen Mittel des ersten Erfindungsgegenstandes nicht gespült und auf der Faser belassen werden.

Die nachfolgenden Beispiele sollen den Gegenstand der vorliegenden Erfindung erläutern ohne ihn in irgendeiner Weise zu beschränken.

Beispiele

Es wurden folgende Handelsprodukte verwendet:

| | |
|---|---|
| Plantacare® 818 UP | C8-14-Alkylpolyglucosid (ca. 51-53% Aktivsubstanzgehalt; INCI-Bezeichnung: Coco-Glucoside, Aqua (Water)) (Cognis) |
| Euxyl® PE 9010 | Mischung aus 90 Gew.-% 2-Phenoxyethanol und 10 Gew.-% 3-(2-Ethylhexyloxy)-1,2-propandiol (100 Gew.-% Aktivsubstanz, INCI-Bezeichnung: Phenoxyethanol, Ethylhexyl Glycerin) (Schülke & Mayr) |

1.0 **Styling Paste zum Modellieren**

Tabelle 1:

| RG | Rohstoffe | Gew.-% |
|---|---|---|
| 1 | Wasser | 56,60 |
| 1 | Natriumhydroxid | 0,50 |
| 1 | Klettenwurzel Extrakt | 0,10 |
| 1 | Sorbitol | 2,10 |
| 1 | Plantacare® 818 UP | 5,00 |
| 2 | Xanthangummi | 0,15 |
| 2 | Dehydroxanthan | 1,20 |
| 2 | Propylenoxid modifizierte Tapiokastärke* | 0,30 |
| 2 | Stearinsäure | 4,00 |
| 2 | Capryl-/Caprinsäure-Triglycerid | 3,00 |
| 2 | Johannisbrotbaummehl | 0,10 |
| 2 | Guar Gummi | 0,05 |
| 2 | Palmitinsäure | 2,00 |
| 2 | Candellilawachs | 3,50 |
| 2 | Isopropylmyristat | 7,00 |
| 2 | Shea Butter | 1,00 |
| 2 | Carnaubawachs | 7,00 |
| 2 | Bienenwachs | 5,00 |
| 3 | Euxyl PE 9010® | 1,00 |
| 3 | Milchsäure | 0,08 |
| 3 | Parfum | 0,50 |

* mit 10 Gew.-% Propylenoxid modifizierte Tapiokastärke, Molmasse 200 bis 300 kDa,

Herstellung:

Schritt I

In einem Gefäß wurden die mit RG 1 gekennzeichneten Rohstoffe der Tabelle 1 vorgelegt. Die Mischung wurde auf 75°C unter Rühren erwärmt.

Schritt II

In einem zweiten Gefäß wurden die mit RG 2 gekennzeichneten Rohstoffe der Tabelle 1 vorgelegt. Die Mischung wurde auf 75°C unter leichtem Rühren erwärmt.

Schritt III

Als beide Mischungen auf 75°C temperiert waren, wurde der Inhalt des ersten Gefäßes unter starkem Rühren in das zweite Gefäß überführt. Nach Beendigung der Zugabe wurde für mindestens 15 Minuten stark weiter gerührt.

Schritt IV

Die mit RG 3 gekennzeichneten Rohstoffe der Tabelle 1 wurden in das zweite Gefäß gegeben und die resultierende Mischung für mindestens 10 Minuten bis zur Homogenität gerührt.
Die resultierende Stylingpaste eignete sich bestens zur Separation einzelner Haarpartien. Es wurde ein Matteffekt erzielt. Die nach Anwendung der Stylingpaste bewirkte Frisur erhielt einen starken Halt und war remodulierbar.

2.0 **Styling Creme zur Bändigung fliegender Haare**

Tabelle 2:

| RG | Rohstoffe | Gew.-% |
|---|---|---|
| 1 | Wasser | 59,5 |
| 1 | Glycerin (pflanzlich) | 5,16 |
| 1 | Plantacare® 818 UP | 4,50 |
| 2 | Shea Butter | 1,50 |
| 2 | Stearinsäure | 4,00 |
| 2 | Palmitinsäure | 4,00 |
| 2 | Sesamöl | 0,10 |
| 2 | Leinsamen Extrakt (in Sonnenblumenöl) | 0,20 |
| 2 | Candellilawachs | 2,00 |
| 2 | Isopropylmyristat | 10,00 |
| 2 | Capryl-/Caprinsäure-Triglycerid | 7,00 |
| 3 | Propylenoxid modifizierte Tapiokastärke** | 0,50 |
| 3 | Dehydroxanthan | 0,50 |
| 4 | Euxyl PE 9010® | 1,00 |
| 4 | Milchsäure | 0,04 |
| 4 | Parfum | 0,50 |

** mit 10 Gew.-% Propylenoxid modifizierte Tapiokastärke, Molmasse 700 bis 900 kDa

Herstellung:

Schritt I

In ein erstes Gefäß wurden die mit RG 2 gekennzeichneten Rohstoffe der Tabelle 2 auf 85 °C unter Rühren erwärmt, bis die Phase homogen wurde.

Schritt II

Ein zweites Gefäß wurde mit den mit RG1 gekennzeichneten Rohstoffen der Tabelle 2 befüllt und die Mischung unter Rühren auf 60°C erwärmt. Es wurde solange gerührt, bis die Mischung homogen war und sich alle Klumpen gelöst hatten.

Schritt III

Die mit RG 3 gekennzeichneten Rohstoffe der Tabelle 2 wurden zu der Mischung des zweiten Gefäßes hinzugefügt und unter starkem Rühren eingearbeitet. Anschließend wurde die Mischung auf 85°C erwärmt und mindestens 20 Minuten bis zur Homogenität gerührt.

Schritt 4

Nachdem beide Mischungen auf 85°C temperiert waren, wurde die Mischung des zweiten Gefäßes zu der Mischung des ersten Gefäßes gegeben. Danach wurde auf 80°C temperiert und über einen Zeitraum von 100 Minuten gerührt.

Schritt 5

Sodann wurde die Mischung unter Rühren auf 40°C abgekühlt und danach die mit RG 4 gekennzeichneten Rohstoffe der Tabelle 2 zugefügt. Abschließend wurde mindestens 15 Minuten bis zur Homogenität rührengelassen und dann die Mischung auf Umgebungstemperatur abkühlen gelassen.

Die resultierende Stylingcreme bändigte störrisches Haar. Die resultierende Frisur erhielt einen guten Halt und glänzte natürlich.

3.0 **Wirkungsnachweis**

Es wurden folgende Propylenoxid modifizierte Stärken verwendet:

Tabelle 1: Propylenoxid modifizierte Stärken

| Bezeichnung der modifizierten Stärke | mittleres Molekulargewicht der modifizierten Stärke (kDa) | Viskosität der modifizierten Stärke [mPas] |
|---|---|---|
| HPS A [1] | 700-900 | 64000 |
| HPS B [2] | 1400-1800 | 170000 |
| HPS C [3] | 200-300 | 6300 |
| HPS D [4] | 700-900 | 55000 |
| [1] Tapiokastärke mit 10 Gew.-% Propylenoxid modifiziert<br>[2] Tapiokastärke mit 10 Gew.-% Propylenoxid modifiziert und vernetzt<br>[3] Tapiokastärke mit 10 Gew.-% Propylenoxid modifiziert und mit Wasserstoffperoxid degradiert<br>[4] Kartoffelstärke mit 10 Gew.-% Propylenoxid modifiziert | | |

Alle modifizierten Stärkederivate ließen sich in Wasser zu 5 Gew.-% bzw. 10 Gew.-% lösen bzw. stabil dispergieren.

Es wurden jeweils 5 Gew.-%-ige Polymerlösungen der jeweiligen Hydroxypropylstärken HPS A bis D und von Polyvinylpyrrolidon/Vinylacetat-Copolymer (PVP/VA) (Luviskol® 64 W NP, Fa. BASF SE) in Wasser hergestellt. Sodann wurden Omega-Loop-Messungen und Messungen der Curl Retention an damit behandelten Haarsträhnen durchgeführt. Zu diesem Zweck wurden Haarsträhnen nach der unter 4.0 und 5.0 beschriebenen Vorgehensweise untersucht.

Folgende Ergebnisse wurden erhalten:

a) Halt, Elastizität und Flexibilität (Omega Loop-Methode)

Tabelle 2: Halt, Elastizität, Flexibilität

| Polymerlösung | F max (N) | Elastizität | Flexibilität |
|---|---|---|---|
| HPS A - 5 Gew.-% | 1,3 | 65% | 68% |
| HPS B - 5 Gew.-% | 1,1 | 34% | 67% |
| HPS C - 5 Gew.-% | 1,3 | 53% | 74% |
| HPS D - 5 Gew.-% | 1,2 | 50% | 70% |
| **PVP/VA - 5 Gew.-%** | **1,1** | **34%** | **67%** |

Je größer die F max-Werte, umso besser ist der Frisurenhalt. Die Werte der Tabelle 2 belegen, dass die erfindungsgemäßen Propylenoxid modifizierten Stärkederivate einen zum gängigen Filmbildner PVP/VA vergleichbaren - bei niederem Molekulargewicht der Propylenoxid modifizierten Stärkederivate sogar besseren - Frisurenhalt ergeben.
Je größer die Werte der Elastizität bzw. der Flexibilität, umso besser ist die Elastizität bzw. die Flexibilität der Frisur. Die mit den erfindungsgemäßen Mitteln behandelten Haarsträhnen wiesen einen flexibleren und elastischeren Halt auf, als die Strähnen die mit PVP/VA-Copolymer behandelt wurden.
Diese Trends blieben bei Einsatz höherer Polymerkonzentrationen (10 Gew.-%) erkennbar.

b) High Humidity Curl Retention (HHCR)

Tabelle 3: Lockenerhalt bei hoher Luftfeuchtigkeit (HHCR)

| Polymerlösung | HHCR |
|---|---|
| HPS A - 5 Gew.-% | 45% |
| HPS B - 5 Gew.-% | 59 % |
| HPS C - 5 Gew.-% | 52 % |
| HPS D - 5 Gew.-% | 75 % |
| **PVP/VA - 5 Gew.-%** | **44%** |

Die mit dem erfindungsgemäßen Mittel behandelten Haarsträhnen wiesen einen gegen Luftfeuchtigkeit beständigeren Halt der Frisur auf.
Dieser Trend blieb bei Einsatz höherer Polymerkonzentrationen (10 Gew.-%) erkennbar.

4.0 **Durchführung der Omega Loop-Messung**

Der Halt der Verformung, auch als Frisurenhalt bezeichnet, sowie Flexibilität und Elastizität werden im Sinne der vorliegenden Erfindung nach der Omega-Loop Methode bestimmt.
Dazu wird eine trockene Haarsträhne (Euro-Naturhaar der Firma Kerling, Klebetresse dicht, einseitig geklebt, Gesamtlänge 160 mm, freie Länge 150 mm, Breite 10 mm, Gewicht 1,0 $\pm$ 0,1 g) für 30 Sekunden bis zum unteren Rand der Abklebung in die zu untersuchende Polymerlösung getaucht. Anschließend wird die überschüssige Lösung zwischen Daumen und Zeigefinger abgestrichen, so dass 0,5 $\pm$ 0,02 g der Lösung auf dem Haar verbleiben. Die mit der zu untersuchenden Lösung gesättigte Haarsträhne wird um einen Teflon-Zylinder mit einem Durchmesser von 36 mm gewickelt und die überstehenden Enden werden mit einem Clip fixiert. Die präparierten Strähnen werden anschließend über Nacht bei 25°C und 50% relativer Luftfeuchte oder bei 25°C und 75% relativer Luftfeuchte im Klimaschrank getrocknet und konditioniert.
Die konditionierte Strähne wird vorsichtig von dem Teflon-Zylinder entfernt. Der entstandene $\Omega$-Loop, eine ringförmige Struktur des in seiner Form durch den ausgebildeten Polymerfilm stabilisierten Haars, wird in den an der Messdose befestigten Greifer eingespannt und bis dicht über die Bodenplatte eines Universalprüfgeräts AMETEK LF Plus der Firma AMETEK Precision Instuments Europe GmbH, Produktgruppe Lloyd abgesenkt. Die gesamte Messung erfolgt im Klimaschrank unter konstanten klimatischen Bedingungen bei 25°C und 50% relativer Luftfeuchte oder bei 25°C und 75% relativer Luftfeuchte.

Um standardisierte Ausgangsbedingungen zu schaffen, startet die Messung mit dem Anfahren einer Vorlast von 0,07 N mit einer Geschwindigkeit von 30 mm $min^{-1}$. Anschließend wird der Ω-Loop mit einer Geschwindigkeit von 60 mm $min^{-1}$ um 8 mm gestaucht, wobei die dazu nötige Kraft gemessen wird. Nachdem die charakteristische Kraft $F_1$ bei der maximalen Deformation von 8 mm aufgezeichnet wurde, wird die Strähne mit 60 mm $min^{-1}$ soweit entlastet, dass sie 10 mm von der Bodenplatte abhebt. Von hier aus beginnt der nächste Zyklus, indem erneut die Vorlast von 0,07 N angefahren und die Strähne anschließend um 8 mm gestaucht wird, hierbei gelten die gleichen Geschwindigkeiten wie oben beschrieben. Die Messung eines Ω-Loops umfasst insgesamt 10 Zyklen.

Mit dieser Messmethode lassen sich vier charakteristische Parameter zur Beschreibung der mechanischen Eigenschaften von filmbildenden Polymeren bestimmen. Halt, Flexibilität, Plastizität und Elastizität lassen sich nach folgenden Formeln aus den gemessenen Kräften berechnen:

$$\mathit{Halt} = F_1\ [N]$$

($F_1$ entspricht der Maximalkraft der Messung)

$$\mathit{Flexibilität} = \frac{F_{10}}{F_1}$$

(gibt das Verhältnis der Maximalkräfte des zehnten zum ersten Zyklus an)

$$\mathit{Elastizität} = \frac{\dfrac{F_{10}(2mm) - F_{10}(1{,}5mm)}{0{,}5}}{\dfrac{F_1(2mm) - F_1(1{,}5mm)}{0{,}5}} = \frac{E_{10}}{E_1}$$

(zur Berechnung der Elastizität werden aus dem ersten und zehnten Zyklus jeweils die Kräfte zur Verformung um 1,5 mm und 2 mm erfasst und miteinander ins Verhältnis gesetzt).

5.0 Durchführung der High Humidity Curl Retention-Messung

Es wurden standardisierte Haarsträhnen der Fa. Kerling (Art. Nr. 827560) des Haartyps "European Natural, Farbe 6/0) von einer Länge ($L_{max}$) von 220 mm und einem Gewicht von 0.6 g eingesetzt. Zur Vorbereitung wurden die Strähnen mit einer 12.5 Gew.-%igen Natriumlaurethsulphat-Lösung gewaschen. Die Haarsträhnen wurden über Nacht in einem Trockenofen bei 318 K getrocknet.

0.18g der Zusammensetzungen wurden auf jeweils eine Haarsträhne appliziert und einmassiert. Die Strähne wurde sodann auf einen Wickler gewickelt (Fripac-medis, ∅ 7mm, Art. Nr. D-1203) und über Nacht bei Raumtemperatur getrocknet.

Die Wickler wurden vorsichtig entfernt und die Strähne aufgehängt. Die Längen der Locken wurden jeweils gemessen ($L_o$) und die Strähnen in eine Klimakammer gegeben. Dort wurden sie bei 294 K und einer relativen Luftfeuchtigkeit von 85% über einen Zeitraum von 24 h gelagert und danach die Längen der Locken erneut vermessen ($L_t$).

Pro Zusammensetzung wurden 5 Teststrähnen entsprechend behandelt und vermessen.

Die High-Humidity-Curlretention (HHCR) wurde nach folgender Formel berechnet und für jede Zusammensetzung das arithmetische Mittel aus den HHCR-Werten der 5 Teststrähnen gebildet:

$$HHCR = \frac{L_{\max} - L_t}{L_{\max} - L_0}$$

## Patentansprüche

1. Kosmetisches Mittel zur temporären Umformung keratinischer Fasern, insbesondere menschlicher Haare, enthaltend in einem kosmetischen Träger mindestens eine mit Propylenoxid modifizierte Stärke, welche ein mittleres

Molekulargewicht (Gewichtsmittel) von 100 bis 1000 kDa, insbesondere von 700 bis 900 kDa, besitzt, wobei die mit Propylenoxid modifizierte Stärke in einer Menge von 2,5 Gew.-% bis 20 Gew.-%, bezogen auf das Gewicht des Mittels, enthalten ist.

2. Kosmetisches Mittel nach Anspruch 1, **dadurch gekennzeichnet, dass** die mit Propylenoxid modifizierte Stärke einen Propylenoxidgehalt von 5 bis 15 Gew.-% - bezogen auf das Gewicht der besagten Stärke - aufweist.

3. Kosmetisches Mittel nach einem der Ansprüche 1 oder 2, **dadurch gekennzeichnet, dass** die mit Propylenoxid modifizierte Stärke einen Propylenoxidgehalt von 8 bis 12 Gew.-% - bezogen auf das Gewicht der besagten Stärke - aufweist.

4. Kosmetisches Mittel nach einem der Ansprüche 1 bis 3, **dadurch gekennzeichnet, dass** das mit Propylenoxid modifizierte Polysaccharid in einer 43 Gew.-%-igen wässrigen Lösung eine Viskosität im Bereich von 150 bis 1500000 mPa s (Brookfield Viskosimeter, Spindel #7 bei 20°C und 20 U/min) aufweist.

5. Kosmetisches Mittel nach einem der Ansprüche 1 bis 4, **dadurch gekennzeichnet, dass** das mit Propylenoxid modifizierte Polysaccharid in einer 43 Gew.-%-igen wässrigen Lösung eine Viskosität im Bereich von 10000 bis 100000 mPa·s (Brookfield Viskosimeter, Spindel #7 bei 20°C und 20 U/min) aufweist.

6. Kosmetisches Mittel nach einem der Ansprüche 1 bis 5, **dadurch gekennzeichnet, dass** die mit Propylenoxid modifizierte Stärke eine mit Propylenoxid modifizierte Tapiokastärke oder eine mit Propylenoxid modifizierte Kartoffelstärke oder einem Gemisch aus beiden vorgenannten Stärken, ist.

7. Kosmetisches Mittel nach einem der Ansprüche 1 bis 6, **dadurch gekennzeichnet, dass** die mit Propylenoxid modifizierte Stärke unvernetzt ist.

8. Kosmetisches Mittel nach einem der Ansprüche 1 bis 7, **dadurch gekennzeichnet, dass** weiterhin mindestens ein zusätzliches Polymer enthalten ist, mit der Maßgabe, dass alle zusätzlichen Polymere Polymere auf Polysacharidbasis sind.

9. Kosmetisches Mittel nach Anspruch 8, **dadurch gekennzeichnet, dass** alle weiteren Polymere ausgewählt werden aus Xanthan, Dehydroxanthan, Alginat, Guar Gum, Gummi Arabicum, Locust Bean Gum, Stärke, Chitosan oder Gemischen.

10. Kosmetisches Mittel nach einem der Ansprüche 1 bis 9, **dadurch gekennzeichnet, dass** es als Schaum oder Gel vorliegt.

11. Kosmetisches Mittel nach einem der Ansprüche 1 bis 10, **dadurch gekennzeichnet, dass** zusätzlich mindestens eine Verbindung der Formel (I) enthalten ist,

$$HO\text{-}CH_2\text{-}(CHOH)_n\text{-}CH_2\text{-}OH \qquad (I)$$

worin n eine ganze Zahl von 1 bis 4 bedeutet.

12. Verwendung eines kosmetischen Mittels nach einem der Ansprüche 1 bis 11 zur temporären Umformung und/oder Formfixierung keratinischer Fasern, insbesondere menschlicher Haare.

13. Verfahren zur temporären Umformung keratinischer Fasern, insbesondere menschlicher Haare, **dadurch gekennzeichnet, dass** ein kosmetisches Mittel nach einem der Ansprüche 1 bis 11 auf die keratinischen Fasern aufgebracht wird.

## Claims

1. A cosmetic agent for temporarily shaping keratinic fibers, in particular human hair, containing in a cosmetic carrier at least one starch modified with propylene oxide which has an average molecular weight (weight average) of from 100 to 1000 kDa, in particular from 700 to 900 kDa, wherein the starch modified with propylene oxide is contained in an amount of from 2.5 wt.% to 20 wt.%, based on the weight of the agent.

2. The cosmetic agent according to claim 1, **characterized in that** the starch modified with propylene oxide has a propylene oxide content of from 5 to 15 wt.%, based on the weight of said starch.

3. The cosmetic agent according to either claim 1 or claim 2, **characterized in that** the starch modified with propylene oxide has a propylene oxide content of from 8 to 12 wt.%, based on the weight of said starch.

4. The cosmetic agent according to one of claims 1 to 3, **characterized in that** the polysaccharide modified with propylene oxide has, in a 43 wt.% aqueous solution, a viscosity in the range of from 150 to 1500000 mPas (Brookfield viscometer, spindle 7 at 20 °C and 20 rpm).

5. The cosmetic agent according to one of claims 1 to 4, **characterized in that** the polysaccharide modified with propylene oxide has, in a 43 wt.% aqueous solution, a viscosity in the range of from 10000 to 100000 mPas (Brookfield viscometer, spindle 7 at 20 °C and 20 rpm).

6. The cosmetic agent according to one of claims 1 to 5, **characterized in that** the starch modified with propylene oxide is a tapioca starch modified with propylene oxide, or a potato starch modified with propylene oxide, or a mixture of the two above-mentioned starches.

7. The cosmetic agent according to one of claims 1 to 6, **characterized in that** the starch modified with propylene oxide is uncrosslinked.

8. The cosmetic agent according to one of claims 1 to 7, **characterized in that** at least one additional polymer is also contained, with the proviso that all additional polymers are polymers based on polysaccharides.

9. The cosmetic agent according to claim 8, **characterized in that** all additional polymers are selected from Xanthan, dehydroxanthan, alginate, guar gum, gum arabic, locust bean gum, starch, chitosan or mixtures.

10. The cosmetic agent according to one of claims 1 to 9, **characterized in that** it is present as a foam or a gel.

11. The cosmetic agent according to one of claims 1 to 10, **characterized in that**, in addition, at least one compound of formula (I) is contained,

$$HO\text{-}CH_2\text{-}(CHOH)_n\text{-}CH_2\text{-}OH \qquad (I)$$

where n is an integer from 1 to 4.

12. The use of a cosmetic agent according to one of claims 1 to 11 for temporarily shaping and/or fixing the shape of keratinic fibers, in particular human hair.

13. A method for temporarily shaping keratinic fibers, in particular human hair, **characterized in that** a cosmetic agent according to one of claims 1 to 11 is applied to the keratinic fibers.

**Revendications**

1. Produit cosmétique pour déformer temporairement des fibres de kératine, en particulier des cheveux humains, contenant dans un vecteur cosmétique au moins un amidon modifié par de l’oxyde de propylène, possédant un poids moléculaire moyen en poids entre 100 et 1000 kDa, en particulier entre 700 et 900 kDa, l’amidon modifié par de l’oxyde de propylène est contenu à hauteur de 2,5 à 20 % en poids rapporté au poids du produit.

2. Produit cosmétique selon la revendication 1, **caractérisé en ce que** l’amidon modifié par de l’oxyde de propylène présente une teneur en oxyde de propylène entre 5 et 15 % en poids rapporté au poids dudit amidon.

3. Produit cosmétique selon la revendication 1 ou 2, **caractérisé en ce que** l’amidon modifié par de l’oxyde de propylène présente une teneur en oxyde de propylène entre 8 et 12 % en poids rapporté au poids dudit amidon.

4. Produit cosmétique selon une des revendications 1 à 3, **caractérisé en ce que** le polysaccharide modifié par de l’oxyde de propylène présente dans une solution aqueuse à 43 % en poids une viscosité entre 150 et 1 500 000

mPa.s (viscosimètre Brookfield, rotor n°7 à 20°C et 20 tr/min).

5. Produit cosmétique selon une des revendications 1 à 4, **caractérisé en ce que** le polysaccharide modifié par de l'oxyde de propylène présente dans une solution aqueuse à 43 % en poids une viscosité entre 10 000 et 100 000 mPa.s (viscosimètre Brookfield, rotor n°7 à 20°C et 20 tr/min).

6. Produit cosmétique selon une des revendications 1 à 5, **caractérisé en ce que** l'amidon modifié par de l'oxyde de propylène est un amidon de tapioca modifié par de l'oxyde de propylène ou un amidon de pomme de terre modifié par de l'oxyde de propylène ou un mélange des deux amidons susmentionnés.

7. Produit cosmétique selon une des revendications 1 à 6, **caractérisé en ce que** l'amidon modifié par de l'oxyde de propylène n'est pas réticulé.

8. Produit cosmétique selon une des revendications 1 à 7, **caractérisé en ce qu'**il contient en outre au moins un polymère supplémentaire, avec pour condition que tous les polymères supplémentaires soient à base de polysaccharides.

9. Produit cosmétique selon la revendication 8, **caractérisé en ce que** tous les polymères supplémentaires sont choisis parmi le xanthane, le déshydroxanthane, l'alginate, la gomme de guar, la gomme arabique, la gomme de caroube, l'amidon, le chitosane ou leurs mélanges.

10. Produit cosmétique selon une des revendications 1 à 9, **caractérisé en ce qu'**il se présente comme une mousse ou un gel.

11. Produit cosmétique selon une des revendications 1 à 10, **caractérisé en ce qu'**il contient en outre au moins un composé de formule (I),

$$HO\text{-}CH_2\text{-}(CHOH)_n\text{-}CH_2\text{-}OH \qquad (I)$$

où n est un entier entre 1 et 4.

12. Utilisation d'un produit cosmétique selon une des revendications 1 à 11 pour déformer temporairement et/ou fixer la forme de fibres de kératine, en particulier des cheveux humains.

13. Procédé de déformation temporaire de fibres de kératine, en particulier de cheveux humains, **caractérisé en ce qu'**on applique un produit cosmétique selon une des revendications 1 à 11 sur les fibres de kératine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS19738866 A